Europäisches Patentamt

(19) European Patent Office

Office européen des brevets

(11) Numéro de publication : **0 046 613**
**B1**

(12) **FASCICULE DE BREVET EUROPÉEN**

(45) Date de publication du fascicule du brevet :
25.07.84

(51) Int. Cl.³ : **C 12 N 11/00**

(21) Numéro de dépôt : **81200880.3**

(22) Date de dépôt : **05.08.81**

(54) **Procédé d'immobilisation de cellules microbiennes globulaires par adhésion à un support solide.**

(30) Priorité : **22.08.80 BE 201826**

(43) Date de publication de la demande :
**03.03.82 Bulletin 82/09**

(45) Mention de la délivrance du brevet :
**25.07.84 Bulletin 84/30**

(84) Etats contractants désignés :
**AT CH DE FR GB IT LI LU NL SE**

(56) Documents cités :
**BE-A- 884 877**
**FR-A- 2 304 326**
**GB-A- 1 514 425**
**GB-A- 2 001 654**
**US-A- 3 821 086**
**US-A- 4 115 198**
**NATURE, volume 261, 20 mai 1976, no. 5557 BASKINGSTOKE (GB) J.F. KENNEDY et al. "Microbial cells living immobilized on metal hydrozides", pages 242-244**

(73) Titulaire : **Région Wallonne représentée par l'Exécutif Régional Wallon**
**11, Boulevard de l'Empereur**
**B-1000 Bruxelles (BE)**

(72) Inventeur : **Rouxhet, Paul Gérard Sciencés Agronomiques**
**Unité de Physico-Chimie Place de l'Université 1**
**B-1348 Louvain-La-Neuve (BE)**

(74) Mandataire : **Blanchart, André**
**Cellule de Gestion des Contrats Technologiques**
**Place Joséphine Charlotte 3 (Boîtes 27, 28)**
**B-5100 Namur (BE)**

EP 0 046 613 B1

# 0 046 613

## Description

La présente invention a pour objet un procédé d'immobilisation de cellules microbiennes globulaires distribuées sous forme d'une couche régulière sur un support solide, par l'intermédiaire de particules colloïdales.

L'invention a également pour objet la couche de cellules microbiennes ainsi obtenues.

On connaît déjà des procédés d'immobilisation de cellules microbiennes, faisant appel à la fixation sur un support, à la formation de flocons ou à l'inclusion dans une matrice. L'immobilisation par fixation, également appelée adsorption ou adhésion, sur un support solide présente par rapport aux autres procédés l'avantage de maintenir un contact direct de l'ensemble de la population microbienne avec le milieu liquide et, en conséquence, de réduire la limitation par les phénomènes diffusionnels du transfert des substances nutritives ou des substances produites par les microorganismes. Elle présente également l'avantage de fournir un système dont la géométrie ne peut pas se modifier au cours de l'utilisation.

Le procédé faisant l'objet du brevet belge 804.502 est relatif à l'obtention de flocons de cellules microbiennes par la formation de chélate entre les cellules et des hydroxydes métalliques. Il présente l'inconvénient de ne pas maintenir un contact direct de l'ensemble de la population microbienne immobilisée avec le milieu liquide. Le procédé de fermentation, suivant la demande de brevet de Grande-Bretagne 1514425, fait appel à l'immobilisation de microorganismes par la formation d'une réaction de chélation entre d'une part le microorganisme et les oxydes hydratés et d'autre part entre le support polyhydroxylé, pouvant provenir éventuellement des souches bactériennes, et les oxydes hydratés. Du fait que les oxydes hydratés sont précipités au contact du support, il ne peut pas y avoir formation d'une couche unique de particules sur les cellules ou le support. Le brevet USA 4.115.198, visant principalement l'immobilisation d'enzymes sur un support mais mentionnant la possibilité d'immobiliser des cellules entières, fait appel à un recouvrement du support par précipitation sur ce dernier d'un oxyde métallique hydraté. Il est souligné dans l'exposé de ce brevet que le mélange des particules de support avec l'oxyde métallique déjà précipité ne donne pas un matériau sur lequel peut se réaliser l'immobilisation ; la fixation des substances biologiquement actives est attribuée à la formation de chélate. Ce procédé présente l'inconvénient de former nécessairement des précipités en présence du support, ce qui exige la manipulation de réactifs chimiques et lie la reproductibilité des résultats à un contrôle rigoureux de la composition chimique de la solution mise en présence du support.

Un des objets de la présente invention est de développer un procédé d'immobilisation des cellules microbiennes par un procédé simple et efficace ne nécessitant pas de modes opératoires compliqués.

La présente invention a pour but de fixer une couche cellulaire unique, distribuée régulièrement sur toute la surface du support, adhérant fortement au support, par l'intermédiaire de particules colloïdales.

Les cellules immobilisées suivant le procédé de la présente invention présentent un degré de viabilité élevé, c'est-à-dire qu'une proportion élevée d'entre elles conserve la capacité de réaliser notamment des réactions enzymatiques et de se reproduire dans un milieu approprié.

Un autre but de l'invention est d'obtenir des complexes stables formés d'une couche cellulaire unique et du support pouvant être utilisés directement dans des réacteurs, par exemple de fermentation. Par complexes stables, on comprend également des complexes de nature différente (support et/ou cellule microbienne) pouvant être combinés et utilisés par exemple sous forme de couches lamellaires multiples. On peut par exemple superposer sur un même support des couches cellulaires immobilisées de différents microorganismes, ou superposer une série de complexes constitués chacun d'un support auquel est fixée une couche cellulaire, les microorganismes étant de nature identique ou différente d'une couche à l'autre ou au sein de la même couche. Toutes les combinaisons sont possibles tout en ne sortant pas du cadre de la présente invention.

L'invention, telle que caractérisée dans les revendications, consiste à traiter, préalablement à l'immobilisation, les cellules ou le support solide par des particules colloïdales, de manière à former une couche régulière de particules colloïdales sur les cellules ou le support solide. Le lien particule colloïdale-support et particule colloïdale-cellule s'explique par une résultante favorable des interactions aux interfaces. Parmi ces interactions, on peut distinguer les interactions électrostatiques (charge-charge et charge-dipôle), les interactions de Van der Waals, les liaisons hydrogène. Etant donné l'importance des interactions électrostatiques charge-charge, le fait que le colloïde aie une charge superficielle opposée à celle de la cellule et à celle du support est un élément très favorable ; il n'est cependant pas indispensable. On peut adsorber sur le support les particules colloïdales formées indépendamment et mettre ensuite les cellules en contact avec le support ainsi traité. On peut adsorber sur les cellules les particules colloïdales formées indépendamment et ensuite adsorber les cellules ainsi traitées sur le support. Par particules colloïdales formées indépendamment, on comprend des particules colloïdales qui ont été obtenues en absence des cellules et du support. Dans le cas d'utilisation d'un support métallique, on peut traiter superficiellement le métal, de manière à former à sa surface une couche régulière de particules colloïdales de ce métal et mettre ensuite les cellules microbiennes en contact avec le support ainsi traité.

Les supports solides utilisés suivant la présente invention comprennent des matériaux solides minéraux et organiques. Le support à utiliser peut être poreux ou non ; pour certaines applications il peut être préférable d'utiliser un support non poreux et ne présentant pas des alvéoles en surface, de manière à ce que toutes les cellules immobilisées puissent être directement exposées au flux d'un liquide. Parmi les composés minéraux, citons l'utilisation de silice, de silicates et d'aluminosilicates, d'oxydes métalliques, de métaux et de leurs alliages etc... On peut utiliser par exemple le verre, une céramique, le laitier, le sable.

Parmi les composés organiques, citons l'utilisation de polymères et/ou copolymères synthétiques, par exemple les polyamides, les polyesters, les polyoléfines, les composés vinyliques.

Les composés minéraux et organiques cités ci-avant ne constituent que des exemples de supports pouvant être utilisés suivant la présente invention et ne sont nullement limitatifs. Pour le choix du support, on tiendra compte à la fois de considérations physiques et mécaniques, économiques et opérationnelles.

Les supports utilisés peuvent se présenter sous des formes très diverses, par exemple de granules, poudre, billes, plaques, tubes, films, membranes, tissus, tricots, fils, fibres, profilés quelconques.

Par cellules microbiennes globulaires, on comprend les microorganismes unicellulaires et les assemblages de microorganismes du type, par exemple, des levures, des bactéries, des microchampignons.

Par couche cellulaire, on comprend une couche cellulaire unique ou monocouche présentant sur la surface du support approximativement une assise cellulaire, qui peut être caractérisée par un nombre de cellules par unité de surface, désigné par le terme densité de cellules immobilisées.

Les particules colloïdales utilisées suivant la présente invention pour le traitement des cellules ou du support solide sont utilisées sous forme d'une suspension, par exemple dans l'eau. Toutefois pour le traitement du support on peut utiliser une suspension en milieu non aqueux, par exemple dans un solvant organique tel qu'un alcool. Parmi les particules colloïdales utilisées on utilisera de préférence celles qui, dans la zone de pH des solutions, soit de la suspension colloïdale, soit de la suspension cellulaire, possèdent une charge superficielle du signe contraire de la charge des cellules et/ou du support. A titre d'exemple, citons les oxydes et hydroxydes métalliques et les colloïdes organiques.

Parmi les hydroxydes métalliques, citons à titre d'exemple l'oxyde et l'hydroxyde de fer et d'aluminium et leurs combinaisons amorphes ou cristallines, la silice, les aluminosilicates.

Parmi les colloïdes organiques, citons à titre d'exemple les suspensions de type latex naturel ou synthétique.

Dans le cas du traitement du support solide par une suspension de particules colloïdales formées indépendamment, les conditions opératoires doivent permettre aux particules colloïdales d'entrer en contact avec le support et de le recouvrir de manière à obtenir une couche régulière de particules colloïdales sur celui-ci. Le moyen le plus simple et non limitatif pour réaliser ce contact est d'utiliser la sédimentation d'une suspension de volume et de concentration suffisantes. L'homme de l'art fera le choix du pH, de la concentration, de la température pour obtenir les effets recherchés.

Suivant une variante de la présente invention on traite superficiellement le support solide constitué d'un métal de manière à former à sa surface une couche régulière de particules colloïdales d'oxydes et/ou d'hydroxyde de ce métal et on met ensuite les cellules microbiennes en contact avec le support ainsi traité.

Divers matériaux métalliques peuvent convenir pour ce traitement superficiel, en vue de former à leur surface des particules colloïdales. A titre d'exemple, citons l'aluminium, le fer, le titane et leurs alliages.

L'homme de l'art fera choix du type de traitement, chimique, électrochimique etc..., en tenant compte de la nature du support métallique en vue d'obtenir cette couche régulière de particules colloïdales. Dans le cas de l'aluminium, on peut créer cette couche unique de particules colloïdales par un simple traitement à la soude caustique, par exemple par utilisation de NaOH (1 N) à une température de 22 °C pendant 5 minutes. On obtient de cette manière une surface matte très régulière. Par ce traitement, on forme à la surface de l'aluminium des particules colloïdales d'oxyde hydraté d'Al.

Suivant cette invention il n'est pas nécessaire d'effectuer un séchage du support, même après la fixation des particules colloïdales formées antérieurement.

Suivant une autre variante du procédé, les cellules microbiennes peuvent être traitées préalablement à leur immobilisation sur un support solide, par une suspension de particules colloïdales. Les particules colloïdales peuvent être introduites dans la suspension cellulaire soit telles quelles, soit sous forme d'une suspension aqueuse ou autre. Eventuellement, les cellules peuvent être dispersées dans la suspension de particules colloïdales. Le temps de contact et les quantités de cellules et de particules colloïdales mises en présence doivent être tels que les cellules puissent être recouvertes d'une couche de particules colloïdales.

L'immobilisation des cellules microbiennes est obtenue par mise en contact de la cellule avec le support, l'un des deux étant couvert de particules colloïdales. Cette immobilisation est obtenue par la mise en présence avec le support d'une suspension aqueuse de cellules microbiennes, d'une concentration suffisante pour réaliser la couche cellulaire. Après un temps de contact de quelques heures et l'élimination par lavage des cellules non fixées, on constate la fixation d'une couche cellulaire sur le support. Cette durée de contact doit être suffisante et est en général d'environ 4 heures. Ceci n'exclut pas des durées plus courtes ou plus longues. Dans le cas d'un temps de contact trop prolongé, par exemple de 24 heures, entre la suspension cellulaire et le support, on constate une proportion de viabilité des cellules plus faible.

0 046 613

Les avantages obtenus grâce à cette invention sont l'obtention d'une assise unique de cellules fixées au support, d'une quantité élevée de cellules immobilisées par unité de surface du support, d'une distribution régulière des cellules sur la surface du support, d'une forte adhésion des cellules au support.

L'immobilisation de cellules en une assise unique et régulière adhérant fortement au support présente l'avantage d'éliminer la diffusion, à travers un lit de cellules, des substances nutritives et des métabolites produits, de permettre l'utilisation d'un flux élevé de liquide au contact des cellules et donc une diminution des limitations diffusionnelles à proximité de celles-ci, de permettre une régénération homogène de toute la population immobilisée, de permettre l'utilisation des cellules immobilisées sur support aussi bien en lit agité qu'en lit fixe. Ces avantages sont particulièrement précieux pour certaines utilisations de ces cellules immobilisées, telles que l'assimilation ou la production de substances de poids moléculaire élevé, la réalisation d'un dispositif d'ensemencement en continu ou d'un dispositif permettant de récolter des cellules de première génération, n'ayant pas donné de cellules filles. Le fait de ne pas impliquer l'utilisation de sels hydrolysables, est également un avantage.

La présente invention sera mieux comprise à l'aide des exemples non limitatifs ci-après.

### Exemple 1

On dispose d'une suspension aqueuse d'hydroxyde d'aluminium préparée suivant Brace et Matijevic (J. Inorganic Nuclear Chemistry, 35, 3641, 1973) ; les particules ont la forme de sphères dont le diamètre moyen est de 0,247 $\mu$m. On verse une suspension aqueuse d'une concentration de 2.0 à 3.0 $10^9$ particules/ml et de pH 9,1 à température ambiante dans un récipient au fond duquel est placée une plaque de verre ; le niveau de la suspension est à 1 cm au-dessus de la surface du verre. On laisse sédimenter les particules d'alumine pendant 12 à 15 heures. Ensuite on lave la lame de verre par un flux laminaire d'eau de 2 mm d'épaisseur. Après 30 minutes de lavage à une vitesse moyenne de 10 cm/sec., on arrive à un nombre stationnaire de particules fixées égal à environ 1,5 $10^9$ particules/cm$^2$, ce qui correspond bien à une couche unique et assez dense de particules d'hydroxyde d'aluminium sur le verre.

La plaque de verre ainsi conditionnée est placée dans un récipient dans lequel on verse une suspension de Saccharomyces cerevisiae d'une concentration de 30 à 50 · $10^6$ cellules/ml, à un pH compris entre 4 et 9 ; le niveau de la suspension cellulaire est à 0.5 cm au-dessus de la surface du verre. Après 3 à 4 heures de contact, on lave la lame de verre dans un flux d'eau de 2 mm d'épaisseur, à une vitesse de 20 cm/sec., pendant 15 à 20 minutes.

.Les cellules immobilisées sont examinées en microscopie optique et comptées. Les cellules immobilisées sont distribuées de manière régulière, formant une couche cellulaire homogène dont la densité est donnée au tableau I.

### Tableau I

| pH initial de la suspension cel-lulaire | Densité de cellules immobilisées (cellules/mm2). |
|---|---|
| 3,9 | 28600 |
| 6,0 | 32800 |
| 8,1 | 30400 |
| 9,1 | 31900 |

Une proportion d'au moins 90 % des cellules immobilisées ne se colorent pas par le bleu de méthylène et gardent donc la capacité de réduire spontanément celui-ci. Les cellules ainsi immobilisées gardent la capacité de se multiplier.

Un essai comparatif a été réalisé dans les mêmes conditions que ci-dessus mais sans traitement du support par des particules colloïdales, les résultats montrent que la densité de cellules immobilisées est inférieure à 600 cellules/mm$^2$. Les cellules sont distribuées de manière irrégulière et ne forment pas une couche cellulaire homogène.

### Exemple 2

On dispose d'une suspension d'hématite ($Fe_2O_3$) préparée suivant Matijevic et Scheiner (J. Colloid Interface Sci., 63, 509, 1978) ; les particules ont la forme de cubes dont les angles sont arrondis et dont la dimension moyenne est de 0,50 $\mu$m. On verse une suspension aqueuse d'une concentration d'au moins 8 · $10^8$ particules/ml, à température ambiante dans un récipient au fond duquel est placée une plaque de verre ; le niveau de la suspension est de 1 cm au-dessus de la surface du verre. Après une attente de 22

4

heures, qui permet aux particules de sédimenter, les plaques sont lavées par un flux laminaire d'eau de 2 mm d'épaisseur. Un lavage à une vitesse moyenne de 41 cm/sec pendant 10 minutes conduit à un nombre stationnaire de particules fixées, égal à environ $4 \cdot 10^8$ particules/cm$^2$, ce qui correspond bien à une couche unique de particules d'hématite serrées les unes contre les autres.

On prépare une suspension de Saccharomyces cerevisiae d'une concentration de $250 \cdot 10^6$ cellules/ml et l'on ajoute le pH à la valeur désirée (4 ou 6). On verse cette suspension dans un récipient dans lequel se trouvent des plaques de verre recouvertes d'une couche d'hématite comme décrit ci-dessus ; le niveau de la suspension est à 1 cm au-dessus de la surface du verre. Après avoir laissé reposer durant 4 heures, on lave les plaques par un flux laminaire d'une solution de même pH que le pH de la suspension cellulaire ; le liquide de lavage a une épaisseur de 2 mm et une vitesse moyenne de 14 cm/sec. ; le lavage est poursuivi durant un temps donné (10, 30 ou 60 minutes).

Les plaques sont examinées en microscopie optique et les cellules fixées sont comptées. Les cellules immobilisées sont distribuées de manière régulière formant une couche cellulaire homogène dont la densité est donnée au tableau II.

La densité de cellules immobilisées est plus faible que dans l'exemple 1, ce qui s'explique par la dimension plus grande des particules d'hématite, qui donne au joint entre les cellules et le support des propriétés différentes.

Tableau II

Immobilisation de Saccharomyces cerevisiae sur du verre tapissé d'une couche de particules d'hématite

| Temps de lavage du dépôt cellu- laire (minute) | pH de la suspen- sion cellulaire mise en oeuvre | Densité de cel- lules immobili- sées (cellules/ mm$^2$). | Proportion des cellules ne se colorant pas au bleu de méthylène (%). |
|---|---|---|---|
| 10 | 4 | 20000 | 80-90 |
|    | 6 | 18000 | 70 |
| 30 | 4 | 20000 | 80-90 |
|    | 6 | 21400 | 70 |
| 60 | 4 | 24800 | 80-90 |
|    | 6 | 20800 | 70 |

Les cellules ainsi immobilisées gardent la capacité de se multiplier. Les résultats du test au bleu de méthylène sont également présentés au tableau II. Il faut souligner que la perte de la capacité de réduire le bleu de méthylène de la part d'une certaine proportion de cellules n'est pas due à leur immobilisation. L'examen des cellules libres conservées dans l'eau pendant 4 heures donne des résultats semblables à ceux rapportés au tableau II.

Exemple 3

Une tôle d'aluminium est dégraissée par une solution de polyéthylène glycol (0,5 %) ; elle est ensuite immergée dans NaOH (IN) pendant 5 minutes à température ambiante puis rincée à l'eau. La tôle ainsi traitée est maintenue au contact d'eau chaude à 98 °C pendant 10 à 15 minutes. On obtient ainsi une surface matte très régulière.

La tôle ainsi conditionnée est placée dans un récipient dans lequel on verse une suspension de Saccharomyces cerevisiae d'une concentration de 30 à $50 \cdot 10^6$ cellules/ml, dont le pH est compris entre 4 et 9. Cette opération et le lavage ultérieur sont réalisés de la manière décrite dans l'exemple 1.

Les cellules immobilisées sont distribuées de manière régulière formant une couche cellulaire homogène dont la densité est donnée au tableau III

Tableau III

Immobilisation de Saccharomyces cerevisiae sur une tôle d'aluminium dégraissée, puis traitée à la soude et à l'eau chaude

| pH initial de la suspension cellulaire. | Densité de cellules immobilisées (cellules/mm²) |
|---|---|
| 4,1 | 34.830 |
| 6,0 | 36.580 |
| 8,0 | 35.210 |
| 9,1 | 35.670 |

Une proportion d'au moins 90 % des cellules immobilisées gardent la capacité de réduire le bleu de méthylène. Les cellules ainsi immobilisées gardent la capacité de se multiplier.

La quantité de cellules immobilisées est plus élevée que dans l'exemple 1, en raison de la texture différente du joint entre la cellule et le support. Un essai comparatif a été réalisé dans les mêmes conditions que ci-dessus, en dégraissant seulement la tôle d'aluminium et en la lavant à l'eau distillée ; les résultats montrent que la densité de cellules immobilisées est négligeable.

## Exemple 4

Une tôle d'aluminium est traitée à la soude (1N) puis rincée à l'eau à 22 °C. La tôle ainsi conditionnée est placée dans un récipient dans lequel on verse une suspension de Saccharomyces cerevisiae à pH compris entre 4 et 9. Cette opération et le lavage ultérieur sont réalisés de la manière décrite à l'exemple 1. On obtient ainsi la fixation de 33 700 cellules/mm², quel que soit le pH.

Les résultats du test au bleu de méthylène et du test de multiplication cellulaire sont les mêmes qu'à l'exemple 3.

## Exemple 5

On dispose de la suspension d'hydroxyde d'aluminium utilisée pour l'exemple 1. On dispose d'une suspension de cellules de Saccharomyces cerevisiae. On mélange une certaine quantité de la suspension d'hydroxyde d'aluminium et une certaine quantité de la suspension cellulaire dans des tubes en polypropylène, de manière à obtenir 40 ml du mélange à une concentration cellulaire de $10^6$ cellules/ml et une concentration d'hydroxyde d'aluminium variable. Les tubes sont ensuite agités pendant un temps donné à température ambiante.

Une partie des particules d'hydroxyde d'aluminium s'adsorbe sur les cellules de levure. Pour mesurer la quantité d'hydroxyde d'aluminium adsorbé on laisse sédimenter les cellules à température ambiante ; on dose les particules restant en suspension par turbidimétrie ; on dose les particules adsorbées par analyse de l'aluminium par adsorption atomique après traitement acide du sédiment de cellules.

Un examen de l'évolution de la quantité fixée en fonction du temps de contact entre les cellules et les particules colloïdales, à pH 6, montre que l'on obtient une valeur stationnaire après 15 heures.

Des expériences répétées pour différentes concentrations initiales en hydroxyde d'aluminium permettent d'obtenir une courbe donnant la quantité de particules colloïdales fixées en fonction de leur concentration en suspension et ce, pour des pH de 4, 6 et 8. Les résultats obtenus montrent que l'on peut fixer 1 580 particules d'aluminium par cellule de levure. Sachant que les cellules utilisées ont un diamètre moyen de 5,7 µm, on peut estimer que la surface moyenne d'une cellule fait $1,02 \cdot 10^{-10}$ m² et que l'adsorption d'une couche serrée de particules d'hydroxyde d'aluminium correspondrait à 1 780 particules par cellule. Les cellules peuvent donc être recouvertes d'une couche de particules colloïdales.

Après ce traitement les cellules peuvent être fixées sur une plaque de verre par une opération de sédimentation suivie d'un lavage des cellules excédentaires.

Les cellules ainsi conditionnées ont gardé la capacité de se multiplier.

## Revendications

1. Procédé d'immobilisation de cellules microbiennes globulaires sur un support solide, caractérisé en ce que, préalablement à l'immobilisation, les cellules ou le support solide sont traités de manière à former une couche unique de particules colloïdales sur les cellules ou le support solide.

2. Procédé suivant la revendication 1, caractérisé en ce que, préalablement à l'immobilisation, les cellules ou le support solide sont traités par des particules colloïdales obtenues indépendamment.

3. Procédé suivant les revendications 1 et 2, caractérisé en ce que les particules colloïdales sont constituées d'oxydes et/ou d'hydroxydes métalliques ou de particules colloïdales organiques.

4. Procédé suivant les revendications 1 à 3, caractérisé en ce que les particules colloïdales sont constituées d'oxyde et/ou d'hydroxyde de fer, d'aluminium.

5. Procédé suivant les revendications 1 à 4, caractérisé en ce que le support solide est choisi parmi les composés minéraux et organiques.

6. Procédé suivant les revendications 1 à 5, caractérisé en ce que le support est un solide siliceux, un métal ou un polymère synthétique.

7. Procédé suivant les revendications 1 à 6, caractérisé en ce que le support solide est du verre, de l'aluminium, du fer et leurs alliages.

8. Procédé suivant les revendications 1 à 7, caractérisé en ce que, préalablement à l'immobilisation, les cellules ou le support solide sont traités par une suspension colloïdale de particules d'oxydes et/ou d'hydroxydes métalliques de manière à former une couche unique de particules colloïdales sur les cellules ou le support solide.

9. Procédé suivant la revendication 1 caractérisé en ce que l'on traite superficiellement le support solide constitué d'un métal de manière à former à sa surface une couche de particules colloïdales d'oxyde et/ou d'hydroxyde de ce métal et l'on met ensuite les cellules microbiennes en contact avec le support ainsi traité.

10. Procédé suivant la revendication 9, caractérisé en ce que le métal est choisi parmi l'aluminium, le fer, le titane et leurs alliages.

11. Procédé suivant les revendications 1 à 10, caractérisé en ce que les cellules microbiennes globulaires proviennent de cellules de Saccharomyces cerevisiae.

12. Cellules microbiennes globulaires immobilisées sur un support solide obtenues suivant les procédés faisant l'objet des revendications 1 à 11.

13. Complexe stable d'une couche unique de cellules globulaires distribuées uniformément sur un support solide, obtenu par l'application des procédés faisant l'objet des revendications 1 à 10.


## Claims

1. Process for immobilising globular microbial cells on a solid support, characterised in that, prior to the immobilisation, the cells or the solid support are treated so as to form a single layer of colloidal particles on the cells or solid support.

2. Process according to Claim 1, characterised in that, prior to the immobilisation, the cells or the solid support are treated with colloidal particles obtained independently.

3. Process according to Claims 1 and 2, characterised in that the colloidal particles are constituted by metal oxides and/or hydroxides or by organic colloidal particles.

4. Process according to Claims 1 to 3, characterised in that the colloidal particles are constituted by aluminium and iron oxide and/or hydroxide.

5. Process according to Claims 1 to 4, characterised in that the solid support is chosen from among the mineral and organic compounds.

6. Process according to Claims 1 to 5, characterised in that the support is a siliceous solid, a metal or a synthetic polymer.

7. Process according to Claims 1 to 6, characterised in that the solid support consists of glass, aluminium, iron and alloys thereof.

8. Process according to Claims 1 to 7, characterised in that, prior to the immobilisation, the cells or the solid support are treated with a colloidal suspension of particles of metal oxides and/or hydroxides so as to form a single layer of colloidal particles on the cells or the solid support.

9. Process according to Claim 1, characterised in that the solid support constituted by a metal is treated superficially so as to form on its surface a layer of colloidal particles of the oxide and/or hydroxide of this metal and then the microbial cells are brought into contact with the support thus treated.

10. Process according to Claim 9, characterised in that the metal is chosen from aluminium, iron, titanium and alloys thereof.

11. Process according to Claims 1 to 10, characterised in that the globular microbial cells originate from cells of Saccharomyces cerevisiae.

12. Globular microbial cells immobilised on a solid support and obtained in accordance with the processes forming the subject-matter of Claims 1 to 11.

13. Stable complex of a single layer of globular cells distributed uniformly on a solid support, obtained by the application of the processes forming the subject-matter of Claims 1 to 10.


## Ansprüche

1. Verfahren zur Immobilisation von kugelförmigen Bakterienzellen auf einem festen Träger, dadurch gekennzeichnet, daß vor der Immobilisation die Zellen oder der feste Träger derart behandelt werden, daß eine einzige Schicht kolloidaler Partikel auf den Zellen oder dem festen Träger gebildet wird.

2. Verfahren nach Anspruch 1, dadurch gekennzeichnet, daß vor der Immobilisation die Zellen oder der feste Träger mit unabhängig gewonnenen kolloidalen Partikeln behandelt werden.

3. Verfahren nach den Ansprüchen 1 und 2, dadurch gekennzeichnet, daß die kolloidalen Partikel aus Metalloxiden und/oder -hydroxiden oder organischen kolloidalen Partikeln bestehen.

4. Verfahren nach den Ansprüchen 1 bis 3, dadurch gekennzeichnet, daß die kolloidalen Partikel aus Oxid und/oder Hydroxid von Eisen und Aluminium bestehen.

5. Verfahren nach den Ansprüchen 1 bis 4, dadurch gekennzeichnet, daß der feste Träger ausgewählt ist aus den mineralen und organischen Verbindungen.

6. Verfahren nach den Ansprüchen 1 bis 5, dadurch gekennzeichnet, daß der Träger ein kieseliger Feststoff, ein Metall oder ein synthetisches Polymer ist.

7. Verfahren nach den Ansprüchen 1 bis 6, dadurch gekennzeichnet, daß der feste Träger aus Glas, Aluminium, Eisen und ihren Verbindungen besteht.

8. Verfahren nach den Ansprüchen 1 bis 7, dadurch gekennzeichnet, daß vor der Immobilisation die Zellen oder der feste Träger mit einer kolloidalen Suspension von Partikeln von Metalloxiden und/oder -hydroxiden behandelt werden, derart, daß eine einzige Schicht kolloidaler Partikel auf den Zellen oder dem festen Träger gebildet wird.

9. Verfahren nach Anspruch 1, dadurch gekennzeichnet, daß der aus einem Metall bestehende feste Träger oberflächenseitig derart behandelt wird, daß auf seiner Oberfläche eine Schicht kolloidaler Partikel des Oxids und/oder Hydroxids dieses Metalls gebildet wird, und daß anschließend die Bakterienzellen mit dem so behandelten Träger in Berührung gebracht werden.

10. Verfahren nach Anspruch 9, dadurch gekennzeichnet, daß das Metall ausgewählt wird unter Aluminium, Eisen, Titan und deren Legierungen.

11. Verfahren nach den Ansprüchen 1 bis 10, dadurch gekennzeichnet, daß die kugelförmigen Bakterienzellen von Zellen der Saccharomyces Cerevisiae stammen.

12. Kugelförmige Bakterienzellen immobilisiert auf einem festen Träger erhalten nach den Verfahren gemäß den Ansprüchen 1 bis 11.

13. Stabiler Komplex einer einzigen Schicht kugelförmiger Zellen, die gleichmäßig auf einem festen Träger verteilt sind, der durch Ausübung von Verfahren gemäß den Ansprüchen 1 bis 10 erhalten ist.